# EUROPEAN PATENT SPECIFICATION

(11) **EP 2 997 344 B1**
(45) Date of publication and mention of the grant of the patent: **09.06.2021**
(21) Application number: 14721829.1
(22) Date of filing: 30.04.2014
(51) Int. Cl.: G01N 1/28

(54) **TISSUE SEPARATION FROM A SAMPLE**
GEWEBETRENNUNG AUS EINER PROBE
SÉPARATION DES TISSUS À PARTIR D'UN ÉCHANTILLON

(30) Priority: 15.05.2013 EP 13167885
(43) Date of publication of application: 23.03.2016
(73) Proprietor: Koninklijke Philips N.V., 5656 AG Eindhoven (NL)
(72) Inventor: WIMBERGER-FRIEDL, Reinhold, NL-5656 AE Eindhoven (NL); JAARTSVELD, Frank Titus Marie, NL-5656 AE Eindhoven (NL); DE WITZ, Christianne Rossette Maria, NL-5656 AE Eindhoven (NL); CLOUT, Danielle Elisa Willemine, NL-5656 AE Eindhoven (NL)
(74) Representative: Philips Intellectual Property & Standards
(86) International application number: PCT/EP2014/058905
(87) International publication number: WO 2014/184005

(56) References cited:
- WO-A1-03/008934
- WO-A1-2005/033668
- US-A- 6 159 681
- US-A1- 2005 250 219
- US-A1- 2012 273 112
- US-B1- 6 194 157

## Description

### FIELD OF THE INVENTION

The invention relates to a method and a sample separation device for the separation of material from a region of interest in a biological sample.

### BACKGROUND OF THE INVENTION

The US 2003/032082 A1 discloses a method for isolating a part of a layer of biological material. Said part is excised with a focused laser beam and then picked up with an adhesive tape which has previously been attached to the sample.

The WO 2005/033668 A1 discloses a method in which a laser cuts tissue to be isolated from a preparation comprising a biological sample and a stabilizing layer. The stabilizing layer may for example be a foil. In another embodiment, an intermediate layer with prefabricated holes is disposed in front of the aperture of a pneumatic gripper.

WO 03/008934 A1 discloses the arrangement of a preparation between a supporting substrate and a cover substrate, wherein a laser cuts a dissecting section out of all three layers. US 2005/250219 A1 describes a similar approach in which a biological material on an object carrier is covered by a film made from a transparent preparation, mixture and/or pure substance, wherein a laser cuts through all three layers. US 2012/273112 A1 discloses a method for producing an analysis tape for body fluids, in which diagnostic auxiliary labels are transferred to a carrier tape.

US 6 159 681 discloses the use of a photoresist layer which is irradiated to expose specific regions of biological material for subsequent analysis.

US 6 194 157 discloses the use of a photoresist layer which is irradiated to cause biological material to become embedded into the changed photoresist. It can then be collected.

### SUMMARY OF THE INVENTION

Based on this background, it would be advantageous to have means that allow for an easy and accurate separation of material from a sample such as a biological specimen.

This object is addressed by a sample separation method according to claim 1. Preferred embodiments are disclosed in the dependent claims.

The invention may make use of a sample separation device according to claim 11 for the separation of material from a region of interest in a biological sample. Said region of interest in the sample will in the following be called "sample-ROI". It may for example correspond to one or more cells or cell fractions with specific features such as a particular color in a staining assay, wherein it is desirable to extract just the material of the sample-ROI for further testing. The sample separation device comprises a "structuring unit" for providing a cover sheet that has an aperture at the sample-ROI.

The cover sheet may in general consist of any solid material that has a (typically flat) shape of sufficient stability and that allows for the manufacturing of an aperture in/through the sheet. The sheet may for example comprise a layer of a synthetic material such as polymer (polyethylene, polypropylene, polyester, polycarbonate, polyamide, PMMA, etc.) or composite material of a polymer binder with filler particles, cross-linked material, like acrylate, polyurethane, silicone, or a laminate of a polymer carrier layer with an adhesive layer, like a pressure sensitive adhesive, gel, monomer mixture. It should be noted that in some examples not forming part of the present invention the cover sheet exists in two states: initially without aperture ("unstructured cover sheet") and finally with the aperture ("structured cover sheet").

The aperture in the cover sheet may in general have any arbitrary shape and size, including simple geometries such as a circle or a rectangle. Moreover, the aperture may comprise several disconnected patches or openings distributed within the area of the cover sheet. The positioning of the aperture "at the sample-ROI" means that the structured cover sheet can be arranged on the given biological sample in such a way that the aperture at least partially overlaps with the sample-ROI of said sample. Preferably, the aperture and the sample-ROI substantially match each other such that (almost) every point of the aperture is located at a corresponding point of the sample-ROI and vice versa. However, any other type of (partial) overlap is allowed, too (e.g. if the aperture covers only a sub-area of the sample-ROI).

The invention relates to a method for the separation of material from a region of interest, called "sample-ROI", in a biological sample. The method comprises the following steps:
a) Production of a cover sheet with an aperture at the sample-ROI, thus enabling access to the sample-ROI while shielding the remainder of the sample.
b) Application of said cover sheet to the sample, wherein this application is done during step a)
c) Removal of sample material from the sample-ROI through the aperture of the cover sheet.

For the production of the cover sheet with an aperture at the sample-ROI in step a), material is deposited from a nozzle at every point of the whole area of the cover sheet except at points of the desired aperture which corresponds to the sample-ROI.

The cover sheet is thus produced with its aperture in one step on the sample (e.g. by printing).

Once a structured cover sheet has been produced, it may optionally be applied sequentially to several samples, particularly to samples having similar sample-ROIs. Such samples may for example be obtained as consecutive slices from the same biopsy.

The method comprises in general form steps that can be executed with a sample separation device of the kind described above. Explanations provided for said device are therefore analogously valid for the method and vice versa.

The sample separation device and the method have the advantage that they allow for an easy and accurate separation of sample material from a region of interest in a biological sample. This is achieved by applying (depositing) a cover sheet with an aperture just at the position of the sample-ROI to the sample, thus enabling access to the sample-ROI while shielding the remainder of the sample. Any appropriate method can then be applied to actively treat material (only) in the sample-ROI. In particular, it is possible to apply an extraction procedure for the extraction of sample material of the sample-ROI to the whole sample surface because said procedure will only be effective in the area not shielded by the cover sheet, i.e. at the aperture/sample-ROI.

In the following, various preferred embodiments of the invention will be described in more detail that can be realized in connection with the above method and sample separation device (even if they are described in detail only for either the method or the device).

The "separation" of material of the sample-ROI generally refers to any process that makes this material distinguishable (with respect to some previously unique aspect or feature) from the remainder of the sample (or vice versa). Due to the separation and by exploiting the created distinction, said material may then for example be processed in-situ separately from the remainder of the sample. The "separation" comprises or entails the removal of sample material from the sample-ROI through the aperture in the cover sheet. The sample separation device may preferably comprise a "removal unit" for this purpose, i.e. for removing sample material from the sample-ROI through the aperture in the cover sheet.

As already explained above, it is comprised by all embodiments of the invention that a structured cover sheet can sequentially be applied to several samples. In the context of the aforementioned embodiment, this has the advantage that enough material can be removed (through the aperture) from said samples for a subsequent processing step.

The cover sheet with the aperture is successively ("point-by-point") built up by depositing material. Thus it can from the beginning be formed with an aperture, i.e. without the need to intermediately produce an unstructured cover sheet.

The successive deposition can particularly be a printing or plotting process. The outcome of these procedures can well be controlled, for example by a digital computer on which data describing the sample-ROI are available.

The deposited material may preferably be altered physically and/or chemically after deposition. Thus its characteristics can be changed to be optimal for the deposition process on the one hand and for its intended usage on the other hand. For example, the viscosity of the material should be low during the deposition process but high afterwards to provide a stable cover sheet. The deposited material may for instance be heated, cooled, exposed to radiation and/or exposed to reagents such as oxygen.

The biological sample will preferably be provided on some kind of carrier such that it can easily be handled. The carrier may particularly be a (microscope-) slide because this is readily available, well standardized to be compatible with many apparatuses, and of course suited for a microscopic inspection of the sample at hand.

A treatment of sample material of the sample-ROI through the aperture in the cover sheet, for example during its removal, may be done in various ways from which a user can choose the most appropriate one in view of the sample at hand and the intended procedures with the sample material. In one particular embodiment, the sample material at the aperture may be exposed to a reagent, particularly a solution such as a buffer solution, be lysed and/or be heated and/or be cooled. Lysis of sample material is possible if the sample comprises one or more biological cells and if molecular diagnostics shall be executed with the extracted material. Appropriate lysis buffers for the extraction of nucleic acids may for example be found in the US 2011/159485 A1.

Heating of the sample material may in general be favorable as it expedites (chemical) reactions. Moreover, heating may be used to melt material, allowing for its removal by flow. Cooling can for instance be applied to bring heated material back to normal temperatures.

The sample may in general comprise a meltable material, particularly a material that melts at moderate temperatures between about 30 °C and about 90 °C, preferably between about 40 °C and about 70 °C. An example of such a material is paraffin, which is often used for the preparation of a biological sample, particularly the preparation of slices of tissue for microscopic investigation. The meltable material can be liquefied by the application of heat during the removal of sample material. Moreover, phase separation may be achieved by melting such a material, allowing for an easy separation of said material from the actual biological material of interest.

In a preferred embodiment the sample is deparaffinized before the application of the cover sheet by immersion in xylene (or an equivalent solvent) followed by xylene displacement with ethanol. This procedure may also be used in the pathology lab to prepare samples for staining.

According to a further embodiment of the invention, a cover may be disposed at the aperture of the cover sheet, particularly during the removal of the sample material. Thus a more or less closed chamber comprising the sample-ROI can be created. The cover may particularly be provided with an inlet for guiding extraction fluid to the sample-ROI and/or with an outlet for draining (liquefied and/or solved) sample material from the sample-ROI. A simple realization of the cover can be achieved with a cover slip as used in microscopy. A more versatile solution may be achieved with a dedicated cover having e.g. an adhesive to create a chamber of a defined volume that facilitates buffer introduction and removal.

In another embodiment of the invention, an actuator may be provided for applying energy to the sample, particularly in the form of electromagnetic radiation, heat and/or ultrasound. The energy may for example be applied during the removal of sample material from the sample-ROI. Thus the detachment of the desired sample material from the sample and/or a substrate (carrier) can be assisted. The actuator may optionally comprise a heater, a light source, and/or an ultrasonic transducer.

In general, the sample-ROI may be selected and defined by any procedure suited for the application at hand. In a preferred embodiment, the sample-ROI is derived from a region of interest in an image of an object related to the sample. Said region of interest in the image will in the following be called "image-ROI" to distinguish it from the sample-ROI. The "object related to the sample" may be the sample itself. It may however also be an additional (sub-) sample generated from the same material ("higher-level sample") as the sample at hand was taken from. It may for instance be a (preferably neighboring) slice generated from the same biopsy as the sample. The image may preferably be a microscopic image, for example generated with a digital scanning microscope. Moreover, the definition of the image-ROI may be done manually, e.g. by a pathologist, or automatically by image analysis software, or by a combination of both.

The aforementioned derivation of the sample-ROI from the selected image-ROI and/or the selection of the image-ROI are preferably executed while taking the capabilities of the cover sheet structuring into account. If cover sheet structuring is for example achieved by a cutting technology, there will typically be lower limits on the spatial resolution and/or the curvature of boundaries. Taking these limits into account will avoid the usage of a sample-ROI or an image-ROI that cannot be realized by the actual structuring procedure.

A given image-ROI may optionally be used to derive more than one sample-ROI. If for example several individual samples are obtained from a single "higher-level sample", for example several slices from a given biopsy, it will usually be necessary to identify the sample-ROI on each individual sample/slice. The image-ROI may then be identical, based on a single reference, however, the position of the sample-ROI and also its shape can differ for each individual slice. Hence there may be several sample-ROIs referring to the same image ROI for the purpose of separating material from consecutive slices originating from the same tissue block. It should be noted that the different sample-ROIs will usually require the production of a plurality of associated cover sheets.

In another embodiment of the invention, an image of the sample may be generated after removal of sample material from the sample-ROI. Such an image may be useful for verifying if the desired material has actually been removed or not. The image may be generated with the cover sheet still in place (taking the image either from the sample side or from the opposite side of the cover sheet) or after removal of the cover sheet.

In another embodiment an image of the sample may be generated before the application of the cover sheet to verify the position and/or deformation of the sample on the slide for an accurate positioning of the aperture of the cover sheet. Optionally an image can be taken after application of the cover sheet to verify the exact position of the aperture before the sample material is removed.

Sample material obtained from the sample-ROI may preferably be subjected to a molecular assay. In this context, the term "molecular assay" is to be understood in a broad sense, comprising any examination, test, or experiment by which one or more parameters of the material from the sample-ROI that depend on its chemical composition may be determined. As an example, the molecular assay may comprise the (qualitative or quantitative) detection of particular proteins or nucleic acid sequences (e.g. tumor markers). In particular, the molecular assay may comprise PCR (e.g. q-PCR, qRT-PCR, RT-PCR, qrt-PCR, or digital PCR), sequencing (particularly next gen sequencing), or micro-array hybridization, or another molecular assay technology or a combination of these.

### BRIEF DESCRIPTION OF THE DRAWINGS

These and other aspects of the invention will be apparent from and elucidated with reference to the embodiments described hereinafter.

In the drawings:
Fig. 1 schematically shows the extraction of sample material;
Fig. 2 illustrates the structuring of a cover sheet by laser cutting;
Fig. 3 illustrates the application of said structured cover sheet to a sample;
Fig. 4 illustrates the removal of sample material from a sample-ROI at the aperture of the cover sheet;
Fig. 5 illustrates the printing of a cover sheet with an aperture;
Fig. 6 illustrates the curing of the printed material of Figure 5;
Fig. 7 shows results of experiments using cover sheets with an aperture for sample extraction.

Like reference numbers refer in the Figures to identical or similar components.

### DETAILED DESCRIPTION OF EMBODIMENTS

Pathology diagnostic investigation of patient material (e.g. tissue and cells) is the basis of many treatment decisions, in particular in oncology. Standard, thin slices from a biopsy are presented on microscope slides and stained according to certain protocols to visualize the morphology of the tissue, e.g. by Hematoxylin-Eosin (short H&E). More recently *in situ* staining for disease-specific biomarkers is being developed for companion diagnostics of targeted drugs, based on the specific binding of antibodies to proteins present on the tissue, so-called immuno-histochemistry (IHC), and hybridization of designed sequences of nucleotides to parts of chromosomes or genes (in-situ hybridization, ISH). Assessment can generally be done with a bright field microscope. Slides can be stored after investigation for a long period as back-up in case the diagnosis needs to be re-assessed.

With increasing understanding of the role of genetic mutations in cancer cells molecular diagnostics ("MDx") are becoming an essential part of pathology for selecting targeted therapies and predicting treatment response. This can be done by q-PCR micro-array and/or sequencing (Sanger or next generation) on the tissue. For the sensitivity and specificity of the MDx analyses it is of essential importance to select only relevant areas of the tissue slice. Dilution by non-cancerous tissue can lead to misdiagnosis.

Presently the pathologist marks with some kind of pen the area that needs to be selected for MDx. A lab technician then tries to remove the selected area from a new (thicker) slice of the same biopsy or even from the tissue block. This procedure is very inaccurate and cumbersome for two reasons: firstly the marking of the section is difficult to do by hand, especially if the image has been acquired by digital scanning. The pathologist needs to translate the image in his head to mark the slide by hand. Secondly, the technician needs to find a way to precisely scratch off the material without losing any or peeling off material outside the selected area. Moreover, contamination of the sample is a problem.

In summary, known procedures for sample extraction have one or more of the following drawbacks:
- Cumbersome manual procedure.
- Inaccurate selection of sample material from slide.
- Risk of contamination due to manual procedure.
- Risk of misdiagnosis due to inaccurate selection.
- Lack of documentation of selected material.
- Lack of standardization and reproducibility.

In the following, an embodiment of the invention will be described that addresses the above issues and presents a new way of selecting tissue sample material from a thin section after histopathological investigation for MDx analysis. One feature of this approach is the wet processing, i.e. the removal of the desired section of the sample by selective dissolution in e. g. a lysis buffer. The undesired material (outside the region of interest ROI) is protected by a structured layer that is laminated on the slide.

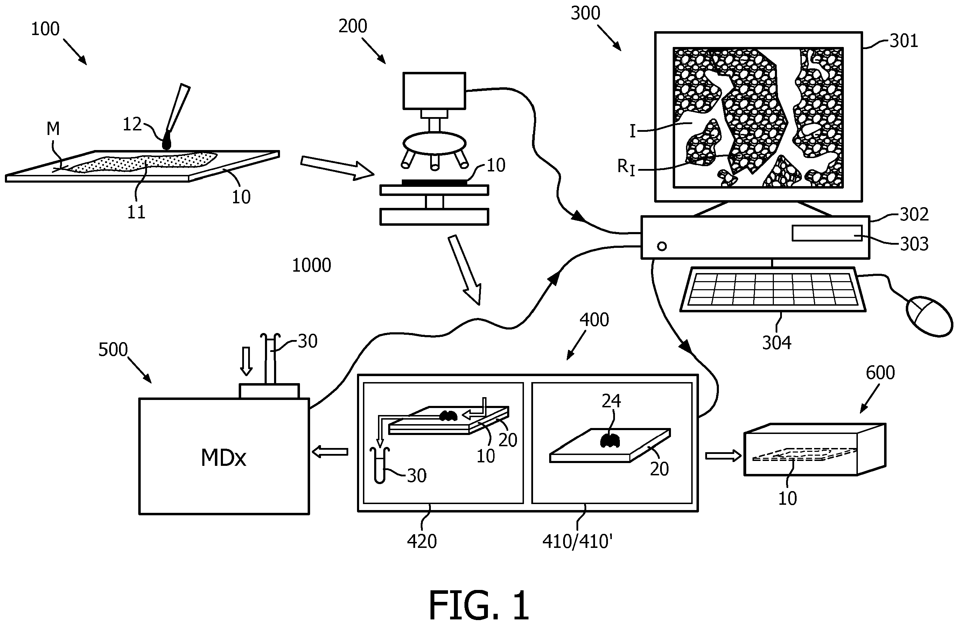

Figure 1 schematically illustrates an approach suited for the examination of a sample 11 of body tissue, not within the scope of the claims. The first step in selective removal of sample from a tissue section is the determination of the region of interest (ROI). This can be done based on histo-pathological staining procedures followed by inspection by a pathologist for diagnosis. Alternatively, the analysis can be supported by image analysis software tools or even completely done automatically. Regions of interest can be characterized according to parameters derived from the image analysis. Depending on the kind of MDx analysis that is required according to clinical protocol the requirements for ROI definition can be adopted. After pathological selection of ROIs additional requirements can be taken into account to arrive at the final design of the ROI. That design is then transferred to a structuring unit, here realized as a tape cutting tool. The cutting tool cuts out a piece of tape that typically has partially exactly the same dimensions as the slide that carries the sample for ease of alignment. The tape is then transferred manually or automatically to the slide in question and is attached to it. These steps will now be described in more detail with reference to Figure 1.

The examination starts at a sample preparation unit 100 in which a slice 11 of body tissue is prepared on a microscope slide 10 serving as a carrier. Typically, tissue samples are obtained by cutting a thin section of about 4-10 microns from a paraffin-embedded biopsy. The so-called coupes are placed on the microscope glass slide 10 on a water film to relax from the micro-toming strain and are then left to dry.

Moreover, the sample 11 may optionally be stained by an appropriate stain 12, e.g. by H&E or IHC. There are a number of staining protocols available for different applications. Staining protocols can be carried out on the bench manually by dipping the slide with the coupe in different solutions containing the reagents, but can also be performed in an automated fashion.

One or more markers M may be printed on or engraved in the microscope slide 10 that can later serve as reference points for relating image coordinates to actual coordinates on the slide 10. Moreover, the slide 10 with the sample 11 may optionally be covered with a cover slip (not shown) in order to allow for a high image quality during later scanning.

After the preparation step, the slide 10 with the sample 11 is transferred to an image generating unit 200, which may particularly be a digital scanning microscope.

A digital image I of the sample is generated by the microscope 200 and communicated to a sample selection unit 300, which is here realized by a workstation 302 with a display (monitor) 301, a memory 303, and input devices 304 like a keyboard and a mouse. The image I of the sample can be displayed on the monitor 301 to allow for a visual inspection by a pathologist. The pathologist can identify a region of interest, R_{I}, in the image and mark it accordingly.

The identification of this "image-ROI" R_{I} may preferably be assisted (or completely be done) by automatic image analysis routines. The area can be optimized for 11 total size etc., optionally taking constraints into account which arise from the sample extraction technology that is later applied (in the sample separation device 400). As a result of the calculation, an image-ROI R_{I} is determined that may be visualized on the screen 301 and/or in the microscope 200 while looking at the sample slide.

The image-ROI R_{I} can then be adjusted manually and selected by the pathologist with the aid of a cursor. Preferably, the pathologist can mark positions in any magnification. The marking will typically be done based on tissue morphology, which is the basis for deciding on the malignancy of the lesion or on the different cell types present. An unlimited number of images can be selected and marked. When finished, the software may provide an overview of the selected image-ROIs in the appropriate magnification for the pathologist and adjust the areas to a necessary resolution which can later be processed by a sample separation device 400 which takes care of the physical extraction of the sample for molecular testing. A file is created that contains the actual (image- and/or sample-) coordinates of the boundaries of the image-ROI R_{I} and the necessary references that can be interpreted by the sample separation device 400.

In the case the actual sample for MDx has been removed from a next slice of tissue and not from the one that was used for image analysis, a translation of the image-ROI has to be made by software using features of the next slice to overlay the image. Such features can be obtained from the he next slice by an optical system optionally after a chemical staining reaction.

The above-mentioned file can be used as input for a sample separation device that is capable of removing the indicated sections directly and to transfer them to a sample holder that can be introduced into the molecular testing equipment (or sample prep equipment).

In the shown example not forming part of the present invention, the microscope slide 10 with the sample 11 is next transferred to the sample separation device 400 in which a sample-ROI, Rs, that corresponds to the selected image-ROI R_{I} is extracted and separated from the remainder of the sample. Preferably, this sample-ROI Rs is transferred to a separate holder, for example a test tube 30. Moreover, the sample separation device may optionally be capable to remove several areas consecutively and submit those to separate molecular tests.

The sample separation device 400 comprises two subunits, namely:
- A "structuring unit" 410 for providing a cover sheet 20 with an aperture 24 at the sample-ROI.
- A removal unit 420 for removing sample material from the sample-ROI through said aperture 24.

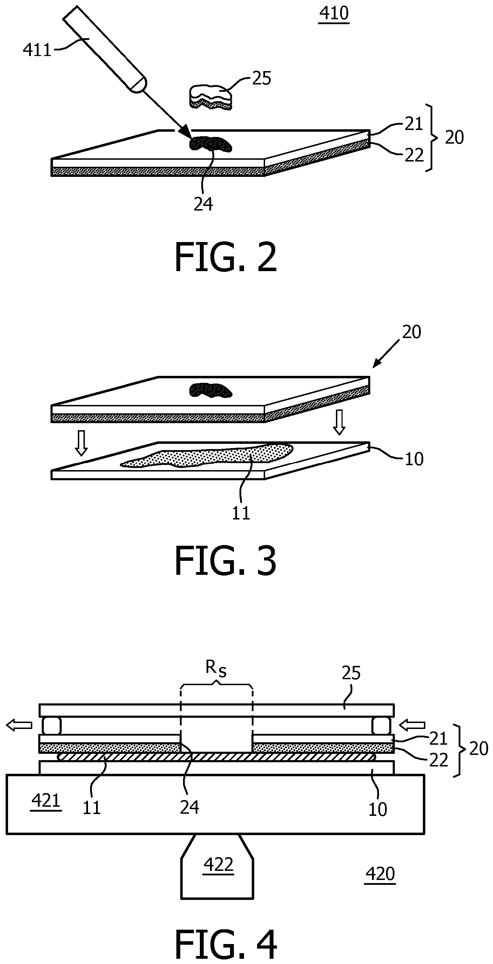

As shown in more detail in Figure 2, the "cover sheet" may be realized by a (commercially available) tape 20, comprising e.g. a carrier sheet 21 of plastic with an adhesive 22 on one side. Accordingly, the structuring unit 410 may be realized by a commercially available automated tape cutter (e.g. a cutting plotter from Roland DG Corporation, Japan). With the structuring unit 410, an aperture 24 corresponding to the desired sample-ROI is generated in the tape 20 by cutting out an associated piece 25 of tape, for example with a laser 411. It should be noted that the aperture 24 may in general have any shape and may particularly consist of several disconnected patches.

As shown in more detail in Figure 3, the structured (pre-cut) tape 20 is laminated (manually or automatically) on the slide 10 with the sample 11, whereby the adhesive 22 attaches to the sample.

As shown in more detail in Figure 4, the slide 10 is then immersed in lysis buffer for dissolving the accessible part of the sample, i.e. the sample-ROI R_{S}. To limit the volume of lysis buffer and for reasons of convenience a preferred example not forming part of the present invention uses a cover 25 that is placed on the tape 20 and has openings (cf. block arrows) to introduce the lysis/extraction buffer that can be closed afterwards.

The slide 20 with the cover 25 may preferably be placed on a hotplate 421 and brought to the desired temperature for complete lysis and/or extraction of nucleic acids and optionally melting of the paraffin in the case the sample is a formalin-fixed paraffin embedded (FFPE) tissue or cytology sample. Upon heating the exposed part of the sample will dissolve and - in the case of a FFPE sample - the paraffin will be phase-separated from the buffer. Upon cooling down the buffer containing the sample can be pipetted off or poured into an Eppendorf test tube 30 or transferred otherwise to a molecular examination unit 500 for purification, amplification, and detection of the desired biomarkers.

In a preferred example not forming part of the present invention (focused) ultrasound may be applied from an ultrasonic transducer 422 to accelerate cell lysis/extraction.

Moreover, the structured cover sheet 20 can be reused to extract sample material from further (consecutive) slices (taken from the same biopsy) with reference to the same image ROI to obtain enough material for a successful MDx test. For example, the same sample-ROI may be removed from up to ten slices from the same tissue block (depending on the number of tumor cells in the ROI and the kind of MDx test).

Additionally or alternatively, one image-ROI may optionally be used to produce several cover sheets related to more than one sample-ROI. If for example several slices are generated from a given biopsy, it will usually be necessary to identify the sample-ROI on each individual slice because e.g. the position of the sample-ROI and also its shape can differ from slice to slice.

A new image may be scanned from the tissue slide 10 before and/or after extraction of the sample-ROI R_{S} to confirm and control the selection. This image may be archived on the workstation 302 together with the original image and the results of the MDx analysis.

The microscope slide 10 with the remainder of the sample can optionally be stored in a storage unit 600 for later access and verification, or it may simply be discarded. Later processing steps with the slide 10 may particularly comprise a follow up examination comprising for example at least one new (particularly different) staining and/or at least one new (particularly different) molecular assay with another region of interest.

In the molecular examination unit 500, several molecular techniques may be available for analysis of the selected sample-ROI, like PCR (several techniques are comprised under this term, like q-PCR, RT-PCR, qrt-PCR, digital PCR, etc.) for detecting single point genetic mutations in cancer cells or any other DNA-mutation, DNA-deletion, DNA-insertion, DNA-rearrangements or copy number amplification or other structural change, and/or determining the degree of RNA expression of genes or other transcribed DNA sequences in cancer cells, or RNA or DNA sequencing (next gen sequencing) for determining a wider spectrum of genetic variations in the cancer cells, for example either on the whole genome, or the whole exome, or targeted to smaller regions of the genome, to the exosome, or the transcriptome, and in various depths. The result is interpreted in terms of genetic mutations of the cancer cells and their corresponding RNA expression profiles which are relevant for the prognosis or alternatively the susceptibility to certain treatment, like by targeted drugs, or to actually assess the effect of a treatment already started or finished (treatment monitoring).

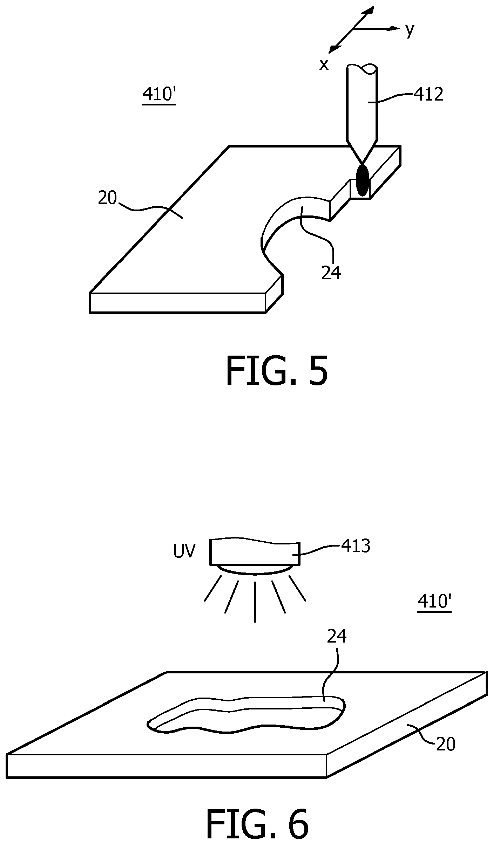

Figures 5 and 6 illustrate an embodiment in accordance with the invention of a "structuring unit" 410' for producing a cover sheet 20 with an aperture 24 that can be used instead of the unit 410 to allow for an automated, flexible and precise selection of tissue from a standard microscopy slide.

The general procedure is similar to the one described above: First, a region of interest is selected as an image-ROI from an image of the sample, preferably based on image analysis and interpretation of stained tissue. The file describing this image-ROI is then transferred to the structuring unit 410' that shall produce a cover sheet 20 with an aperture 24 corresponding to the image-ROI (and, after application to the sample, to the sample-ROI).

The projection of the determined image-ROI in the structuring unit 410' requires alignment. There are two main options of achieving that, depending on whether the identical slide is used that was used for the determination of the image-ROI. In that case mechanical alignment can be used without additional imaging. In the case of a new slide with the neighboring slice of tissue, the new slide needs to be imaged by the structuring unit 410'.

The main novel step is that the protective cover sheet 20 is produced by a material-depositing technology such as printing or plotting.

Figure 5 illustrates this for the case of printing. A nozzle 412 of a printer is moved sequentially row-by-row in x- and y-direction to cover the whole area of the cover sheet 20 to be produced. Material is deposited from the nozzle at every point except at points of the desired aperture 24 which corresponds to the image/sample-ROI.

Different printing techniques are available for this step as there are different inks available. The main requirement for the deposited material is that is must withstand the conditions of separation of the sample material in the aperture, i.e. not dissolve in a buffer at elevated temperatures (depending on protocol this can be up to 80 °C) and elevated pH. Good adhesion to the substrate and the tissue is required as well. Typically, a cover or cap is applied on the cover sheet to create a volume for the convenient introduction of the separation buffer (cf. Figure 4). The adhesion of the cap on the protective cover sheet is required as well. For ease of control of the alignment it is beneficial to have a clearly visible color of the cover sheet 20.

Most printing techniques, e.g. inkjet, require a low viscosity of the ink. This can be achieved in different ways, for example:
(i) heating a high viscosity material,
(ii) using a low viscosity precursor material and converting that after printing, or
(iii) making a solution of the material in a low viscosity solvent and removing the solvent after printing.

The cover sheet 20 can optionally be further treated after printing for stabilization by e.g. irradiation, heating or combinations. Moreover, it is generally desirable to have an easy-to-use solution for pathology. Therefore embodiments are preferred that do not require environmental protection due to usage of dangerous solvents or materials.

In one preferred embodiment that meets the above considerations hot melt ink is used for printing. This requires a heated print nozzle (412) and ink supply, but no post printing treatment (besides the cooling that occurs anyway). Materials for this purpose comprise for example wax, ABS polymer and/or PLA polymer.

In another preferred embodiment a UV curable ink is used for printing. After printing the cover sheet is exposed to UV or UV-VIS radiation while the print head is protected from exposure to that radiation. Figure 6 illustrates this for an exposure of the printed cover sheet 20 to UV-irradiation from an UV lamp 413. Materials for this purpose comprise for example epoxides, acrylates, enoles, and/or silicones.

In another preferred embodiment a water-curable ink may be used that solidifies by exposure to humidity. Materials for this purpose comprise for example silicones.

In another embodiment two reactive components may be printed separately and react once deposited on the slide to form a stable layer. Materials for this purpose comprise for example epoxide and amine, isothiocyanate and amine, anhydride and amine, and/or enol and amine.

In another embodiment a polymer solution is printed and left to dry to vitrify. Such systems preferably use environmentally friendly solvents, like water, methanol, ethanol, etc. The system can be provided with an active carbon filter or a condensation reservoir to collect the solvent in case it cannot be evaporated in the surrounding. The quantities of required solvent are very low (typically the printed volume will be less than 0.1 ml per sample and a typical number of samples will be less than 10 per lab and day, leading to an estimated load of less than 1 ml of solvent per day).

Multi-nozzle printers can be employed, drop on demand or continuous drop printers. As an alternative to printing, plotting can be used. In that case a continuous flow of material is provided and the plotting head is moved to cover the required surface. This allows the use of higher viscosity materials (solutions and/or reactive mixtures, and/or hot melts).

The design of the aperture 24 is preferably done by a computer program taking into account the sample-ROI and design constraints from the printing technology. The aperture containing cover sheet 20 is produced without a mask and therefore does not require any preparation, cost or time. Any shape can be created at sub-mm resolution.

The usage of the cover sheet 20 produced in the structuring unit 410' is as described above: After application of the structured cover sheet to the sample, the tissue material is exposed to a solution that dissolves the biomolecules in the sample-ROI (inside the aperture) but not from outside the sample-ROI as this is covered by the protective layer of the cover sheet. The buffer with the biomolecules is then provided for molecular diagnostic analysis, as for instance after optional further purification, amplification and detection by PCR, sequencing or other molecular diagnostic technique.

Optionally a cover can be applied/glued on the slide to confine the lysis solution or buffer which can be applied and removed through access ports in the cover by pipettes. Alternatively, more sophisticated and dedicated embodiments of the lysis and/or extraction step can be used.

After extraction of the sample material the remaining sample can be discarded or alternatively the cover sheet can be stripped off with an appropriate treatment and another cover sheet can be produced with a different aperture for a different analysis. Since there is no solution of the protective layer in the separation buffer no interference with the molecular analysis occurs.

The described approach is very simple and robust. It avoids open, mechanical, and manual removal by scratching which can lead to contamination. The approach can be applied in molecular pathology, in particular for patient stratification in oncology based on identification of molecular changes in cancer cells.

In summary, an embodiment of the invention has been described as an example that allows a reliable and convenient, preferably automated selection of sample material from tissue slides with a thin section after histopathological investigation for molecular diagnostic analyses, like qPCR and/or sequencing. One feature is the wet processing, i.e. the removal of the desired section of the sample by selective dissolution in a lysis buffer. The method is based on covering all sample material except for the part that is selected for analysis. The desired pattern is created by a software tool that allows selection of areas on the image obtained from the sample section before, or from a neighboring slice that has been stained accordingly. The region of interest (ROI) may particularly be selected based on image analysis and interpretation of stained tissue. The ROI data is transferred to a device that provides a cover sheet with an aperture corresponding to the ROI (transposed on the sample slice in the case of using a different slice of sample).

The structuring of a tape can be carried out by a commercially available automated tape cutter. Alternatively, a cover sheet may be provided by printing or plotting a material that forms a continuous layer on the sample outside the ROI. The application of the material can be done by inkjet-, continuous jet-, or 3D printing or similarly by plotting. The material can optionally be a precursor material that is converted after application into a more stable material by UV irradiation, baking or drying. Typical materials can be thermoplastic polymers, monomers or mixtures of non-reactive with reactive components.

From the not covered (ROI) part nucleic acids (or other biomarkers) are dissolved in lysis buffer in a closed device at elevated temperature. FFPE material can be used without pre-treatment or after deparaffination. After lysis the dissolved material can be removed from the device and transferred to the desired analytical instrument or alternatively the device can be coupled directly via a dedicated interface. The sample material can be transferred for further purification, amplification and detection by PCR, sequencing or other molecular diagnostic technique.

The areas outside the region of interest are covered by a layer (tape) that is structured, e.g. by a cutting tool that automatically cuts out the selected areas from a continuous layer, based on the selection done by a pathologist or an automated software interpretation system and applies the layer on the sample slice aligning the aperture to the identified ROI. The lab technician only has to place the cover and introduce the lysis buffer. The method can be applied directly on the investigated tissue sections on a standard glass slide. Inspection after selection can be done readily. The resolution is determined by the tape structuring technology, which can inter alia be lithographic for high resolution and a cutting tool for medium resolution. The system can be linked to a digital pathology scanner and image analysis software for selection of the areas of interest and characterization of the input material.

### Experimental results

The described approach was tested in an experiment for selective extraction of mRNA from FFPE cancer cells SKBR3 using the RNA extraction according to the assay miRNeasy FFPE of Qiagen. The following protocol was applied:
- Cut four sections of 4 µm thick Formalin Fixed-Paraffin-embedded SKBR3 cells from a block.
- Deparaffination: 3 × xylene 8 min; 100% EtOH 5 min & 30 sec
- Application of black tape (Metamark E-series, UK) containing an aperture.
- Application of hybridization chamber on the black tape.
- Extraction: Inject 240 µl PKD + 50 µl proteinase K into 100 µl/sample, close the chamber; keep 15 minutes 60°C, then 15 minutes 80°C.
- Take fluid out of the hybridization chamber and add 100 ul PKD
- RNA purification, cDNA reaction, and PCR.

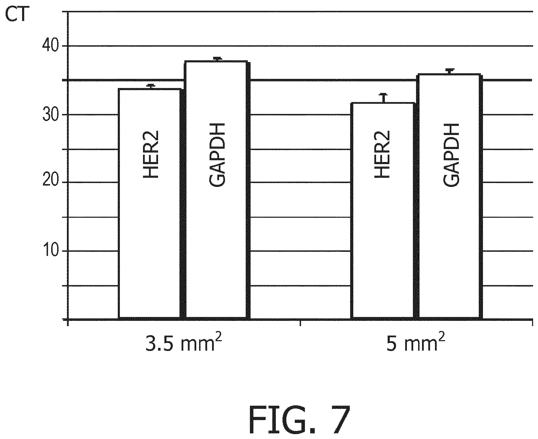

Figure 7 shows the results in terms of threshold cycle number (CT value, vertical axis) for HER2 mRNA and GAPDH mRNA (housekeeping gene) for two sizes of the aperture in the tape, i.e. 3.5 mm² and 5 mm², respectively.

Comparison reveals the following difference: Relative ratio in mRNA copy numbers: 2.23 (converted from CT difference in PCR); surface ratio of aperture: 2.04 (5×5/3.5×3.5).

This yields an estimated number of SKBR cell nuclei in the 3.5×3.5 mm² aperture of section (derived from staining) of 4·10³ cells.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method for the separation of material from a region of interest, called sample-ROI (R_{S}), in a biological sample (11), said method comprising the steps:
a) production of a cover sheet (20) with an aperture (24) at the sample-ROI thus enabling access to the sample-ROI while shielding the remainder of the sample,
b) application of said cover sheet (20) to the sample (11) during step a); and
c) removal of sample material from the sample-ROI through the aperture (24) of the cover sheet (20),
**characterized in that** for the production of the cover sheet (20) with the aperture (24) at the sample-ROI in step a), material is deposited from a nozzle at every point of the whole area of the cover sheet (20) except at points of the desired aperture (24) which correspond to the sample-ROI.

2. The method according to claim 1, wherein the cover sheet (20) is successively built up by depositing material using a printing or plotting process.

3. The method according to claim 2, wherein the deposited material is physically and/or chemically altered after deposition, particularly by heating, cooling, exposure to radiation and/or exposure to reagents.

4. The method according to claim 1, wherein the sample (11) is disposed on a carrier, particularly on a microscope slide (10).

5. The method according to claim 1, wherein the sample material at the sample-ROI (R_{S}) is heated, cooled, exposed to a reagent, particularly a solution such as a buffer solution, and/or lysed.

6. The method according to claim 1, wherein a cover is disposed above the aperture (24).

7. The method according to claim 1, wherein an actuator (422) is provided for applying energy such as electromagnetic radiation, heat and/or ultrasound to the sample.

8. The method according to claim 1, wherein the sample-ROI (Rs) is derived from a region of interest, (R_{I}), in an image (I) related to the sample, particularly an image of the sample itself or of an additional sample generated from the same material as the sample.

9. The method according to claim 1, wherein an image of the sample (11) is generated before and/or after removal of sample material from the sample-ROI (R_{S}).

10. The method according to claim 1, wherein a molecular assay is executed with sample material obtained from the sample-ROI (Rs).

11. A sample separation device (400) for the separation of material from a region of interest, called sample-ROI (R_{S}), in a biological sample (11), comprising:
a structuring unit (410, 410') for providing a cover sheet (20) having an aperture (24) at the sample-ROI, thus enabling access to the sample-ROI while shielding the remainder of the sample and through which sample material from the sample-ROI can be removed.,
**characterized in that** the structuring unit comprises a nozzle for printing or plotting, and **in that** the device is adapted to control the nozzle to deposit material from the nozzle at every point of the whole area of the cover sheet (20) except at points of the desired aperture (24) which correspond to the sample-ROI, thereby providing the cover sheet (20) having the aperture (24) at the sample-ROI.

## Patentansprüche

1. Eine Methode zum Trennen von als Proben-ROI (Rs) bezeichnetem Material aus einer zu untersuchenden Region in einer biologischen Probe (11), wobei die Methode folgende Schritte umfasst:
a) Erzeugen einer Abdeckfolie (20) mit einer Öffnung (24) am Proben-ROI, um den Zugang zum Proben-ROI zu ermöglichen, während der Rest der Probe abgeschirmt wird,
b) Aufbringen der Abdeckfolie (20) auf der Probe (11) im Rahmen von Schritt a); und
c) Entfernen des Probenmaterials aus dem Proben-ROI durch die Öffnung (24) der Abdeckfolie (20),
die sich dadurch auszeichnet, dass für das Erzeugen der Deckfolie (20) mit einer Öffnung (24) am Proben-ROI in Schritt a) mithilfe einer Düse an allen Punkten der gesamten Abdeckfolienfläche (20) mit Ausnahme der Punkte an der gewünschten, der Proben-ROI entsprechenden Öffnung (24) Material aufgebracht wird.

2. Die Methode gemäß Anspruch 1, wobei die Abdeckfolie (20) nach und nach durch Aufbringen von Material mithilfe einer Druck- oder Auftragungsmethode aufgebaut wird.

3. Die Methode gemäß Anspruch 2, wobei das aufgebrachte Material nach dem Aufbringen physikalisch und/oder chemisch verändert wird, insbesondere durch Erwärmung, Abkühlung, Bestrahlung und/oder Auftragen von Reagenzien.

4. Die Methode gemäß Anspruch 1, wobei die Probe (11) auf einem Träger und hierbei insbesondere auf einem Objektträger (10) platziert wird.

5. Die Methode gemäß Anspruch 1, wobei das Probenmaterial in der Proben-ROI (Rs) erwärmt, gekühlt, einer Reagenz und hierbei insbesondere einer Lösung wie z. B. einer Pufferlösung ausgesetzt und/oder lysiert wird.

6. Die Methode gemäß Anspruch 1, wobei sich oberhalb der Öffnung (24) eine Abdeckung befindet.

7. Die Methode gemäß Anspruch 1, wobei ein Aktor (422) die Probe mit Energie z. B. in Form von elektromagnetischer Strahlung, Wärme und/oder Ultraschall beaufschlagt.

8. Die Methode gemäß Anspruch 1, wobei die Proben-ROI (Rs) aus einer zu untersuchenden Region (R_{I}) in einem Bild (I) der Probe abgeleitet wird, insbesondere einem Bild der Probe selbst oder einer zusätzlichen Probe, die aus dem gleichen Material wie die Probe besteht.

9. Die Methode gemäß Anspruch 1, wobei vor und/oder nach dem Entnehmen von Probenmaterial aus der Proben-ROI (Rs) ein Bild der Probe (11) erzeugt wird.

10. Die Methode gemäß Anspruch 1, wobei an dem aus der Proben-ROI (Rs) entnommenen Probenmaterial eine molekulare Untersuchung durchgeführt wird.

11. Ein Probentrenngerät (400) zum Trennen von als Proben-ROI (Rs) bezeichnetem Material aus einer zu untersuchenden Region in einer biologischen Probe (11), wobei das Gerät Folgendes umfasst:
eine Strukturierungseinheit (410, 410') zum Bereitstellen einer Abdeckfolie (20) mit einer Öffnung (24) am Proben-ROI, um den Zugang zum Proben-ROI und das Entnehmen von Probenmaterial aus der Proben-ROI zu ermöglichen, während der Rest der Probe abgeschirmt wird,
das sich dadurch auszeichnet, dass die Strukturierungseinheit eine Düse zum Drucken oder Auftragen umfasst, und dass das Gerät die Düse steuert, um an allen Punkten der gesamten Abdeckfolienfläche (20) mit Ausnahme der Punkte an der gewünschten, der Proben-ROI entsprechenden Öffnung (24) Material aufzutragen, sodass die Abdeckfolie (20) mit einer Öffnung (24) an der Proben-ROI bereitgestellt wird.

## Revendications

1. Procédé de séparation de matière d'une zone d'intérêt, appelée ROI d'échantillon (Rs), dans un échantillon biologique (11), ledit procédé comprenant les étapes suivantes :
a) la production d'une feuille de couverture (20) comportant une ouverture (24) au niveau de la ROI d'échantillon permettant ainsi d'accéder à la ROI d'échantillon tout en protégeant le reste de l'échantillon ;
b) l'application de ladite feuille de couverture (20) à l'échantillon (11) dans l'étape a) ; et
c) l'enlèvement de la matière d'échantillon de la ROI d'échantillon à travers l'ouverture (24) de la feuille de couverture (20),
**caractérisé en ce que** pour la production de la feuille de couverture (20) comportant l'ouverture (24) au niveau de la ROI d'échantillon dans l'étape a), la matière est déposée à partir d'une buse à chaque point de toute la surface de la feuille de couverture (20) sauf aux points de l'ouverture (24) souhaitée, lesquels correspondent à la ROI d'échantillon.

2. Procédé selon la revendication 1, dans lequel la feuille de couverture (20) est formée successivement par dépôt de la matière à l'aide d'un processus d'impression ou de traçage.

3. Procédé selon la revendication 2, dans lequel la matière déposée est physiquement et/ou chimiquement modifié après le dépôt, en particulier par chauffage, par refroidissement, par exposition à un rayonnement et/ou par exposition à des réactifs.

4. Procédé selon la revendication 1, dans lequel l'échantillon (11) est disposé sur un support, en particulier sur une lame de microscope (10).

5. Procédé selon la revendication 1, dans lequel la matière d'échantillon au niveau de la ROI d'échantillon (Rs) est chauffée, refroidie, exposée à un réactif, en particulier une solution telle qu'une solution tampon, et/ou lysée.

6. Procédé selon la revendication 1, dans lequel un couvercle est disposé au-dessus de l'ouverture (24).

7. Procédé selon la revendication 1, dans lequel un actionneur (422) est fourni pour appliquer une énergie, telle qu'un rayonnement électromagnétique, une chaleur et/ou des ultrasons, à l'échantillon.

8. Procédé selon la revendication 1, dans lequel la ROI d'échantillon (Rs) est dérivée d'une zone d'intérêt, (R_{I}), dans une image (I) liée à l'échantillon, en particulier une image de l'échantillon lui-même ou d'un échantillon supplémentaire généré à partir de la même matière que l'échantillon.

9. Procédé selon la revendication 1, dans lequel une image de l'échantillon (11) est générée avant et/ou après l'enlèvement de la matière d'échantillon de la ROI d'échantillon (Rs).

10. Procédé selon la revendication 1, dans lequel un essai moléculaire est exécuté avec la matière d'échantillon obtenue à partir de la ROI d'échantillon (Rs).

11. Dispositif de séparation d'échantillon (400) destiné à la séparation de matière d'une zone d'intérêt, appelée ROI d'échantillon (Rs), dans un échantillon biologique (11), comprenant :
une unité structurale (410, 410') destinée à la fourniture d'une feuille de couverture (20) comportant une ouverture (24) au niveau de la ROI d'échantillon, permettant ainsi l'accès à la ROI d'échantillon tout en protégeant le reste de l'échantillon, et à travers laquelle la matière d'échantillon de la ROI d'échantillon peut être enlevée,
**caractérisé en ce que** l'unité structurale comprend une buse destinée à l'impression ou au traçage, et que ledit dispositif est conçu pour commander la buse pour déposer la matière de la buse à chaque point de toute la surface de la feuille de couverture (20) sauf aux points de l'ouverture (24) souhaitée, lesquels correspondent à la ROI d'échantillon, fournissant ainsi la feuille de couverture (20) comportant l'ouverture (24) au niveau de la ROI d'échantillon.
